# FASCICULE DE BREVET EUROPEEN

(11) **EP 3 420 638 B1**
(45) Date de publication et mention de la délivrance du brevet: **28.04.2021**
(21) Numéro de dépôt: 17707370.7
(22) Date de dépôt: 26.01.2017
(51) Int. Cl.: H03K 17/96, A61B 5/053, A61F 4/00, A61M 21/00, G06F 3/01, A63B 71/00

(54) **UTILISATION DE LA MODULATION D'UN SIGNAL PAR UNE IMPÉDANCE DE CONTACT DE LA PEAU POUR L'ENTRETIEN ET LE DÉVELOPPEMENT DE CAPACITÉS PHYSIQUES OU PSYCHIQUES**
VERWENDUNG DER MODULATION EINES SIGNALS DURCH EINE HAUTKONTAKTIMPEDANZ ZUR ERHALTUNG UND ENTWICKLUNG DER KÖRPERLICHEN ODER GEISTIGEN FÄHIGKEITEN
USE OF THE MODULATION OF A SIGNAL BY A SKIN CONTACT IMPEDANCE FOR THE MAINTENANCE AND DEVELOPMENT OF PHYSICAL OR MENTAL ABILITIES

(30) Priorité: 26.01.2016 FR 1650603
(43) Date de publication de la demande: 02.01.2019
(73) Titulaire: Royer, Philippe, 75019 Paris (FR)
(72) Inventeur: Royer, Philippe, 75019 Paris (FR)
(74) Mandataire: Cornuejols, Georges
(86) Numéro de dépôt international: PCT/FR2017/050181
(87) Numéro de publication internationale: WO 2017/129909

(56) Documents cités:
- WO-A1-86/01317
- US-A1- 2011 306 303
- US-A1- 2013 211 277

## Description

### DOMAINE TECHNIQUE DE L'INVENTION

La présente invention vise une utilisation de la modulation d'un signal par une impédance au contact de la peau pour l'entretien et le développement de capacités physiques et psychiques et, plus particulièrement, une utilisation de la modulation d'un signal audio et/ou visuel par une variation d'impédance au contact de la peau d'au moins un utilisateur pour l'entretien et/ou le développement de ses capacités physiques et/ou psychiques. Elle s'applique, notamment, à l'accompagnement médical de patients en situation d'isolement.

### ETAT DE LA TECHNIQUE

Les personnes en situation de précarité peuvent être victimes d'isolement physique, psychique, sensible et social. Pour contrevenir à cet isolement, des personnels spécialisés accompagnent ces personnes à travers des exercices visant à exercer la créativité et la capacité de communication.

Les exercices actuels visent à favoriser la synesthésie, c'est-à-dire l'association d'un sens à un autre.

Cependant, il n'existe aucun système actuel permettant à une personne seule d'exercer sa créativité dans le contrôle d'un média à partir de son seul sens du toucher et sans actionnement d'une interface homme-machine matérielle, tel un écran tactile par exemple.

De plus, aucun de ces systèmes actuels ne permettent de compenser un handicap ou d'amplifier la mobilisation cérébrale de patients.

On connaît des dispositifs décrits dans les documents WO 2005/121939, US 2012/0262369, US 2015/0177891, US 2011/0134074, US 2011/0306303 et US 2013/0002544. Ces dispositifs sont des télécommandes pour commander un ordinateur ou un appareil électronique avec des capteurs positionnés sur la peau d'un utilisateur. Ils ne permettent aucunement d'entretenir ou de développer des capacités physiques ou psychiques, et encore moins, de mettre en relation électrique au moins deux personnes dans ce but.

### OBJET DE L'INVENTION

La présente invention vise à remédier à tout ou partie de ces inconvénients. A cet effet, la présente invention vise une utilisation selon la revendication 1.

Grâce à ces dispositions, il est possible de générer pour chaque utilisateur un phénomène de synesthésie associant le toucher à un autre sens, tel l'ouïe ou la vue par exemple. Les zones du corps les plus réactives sont celles où les mécanorécepteurs sont les plus nombreux, c'est-à-dire les mains et les pieds par exemple. Cette géographie corporelle permet de jouer de la pression et de la surface de connexion et des zones de contact pour générer des données. La durée de connexion fait augmenter la chaleur et donc la valeur du paramètre mesuré. L'humidification ou l'isolation change également l'impédance.

Ces dispositions permettent ainsi de créer des liens pour répondre à l'isolement physique, psychique, sensible et social des personnes en situation de précarité :
- pour les personnes aphasiques, ces dispositions permettent de communiquer,
- pour les personnes handicapées physiques ou polyhandicapées, ces dispositions agissent comme outil de création, révélateur de compétences, par le contact avec le plus petit morceau de peau,
- pour les personnes âgées, ces dispositions permettent de mobiliser la mémoire consciente et inconsciente à travers le média lu et le toucher,
- pour l'ensemble des personnes à mobilité réduite, ces dispositions permettent la mise en mouvement du corps, la stimulation des mécanorécepteurs et
- pour tout autre type d'utilisateur, ces dispositions permettent une pratique somatique qui apporte relaxation en concentrant les sens.

Des personnels spécialisés accompagnent des personnes en situation de précarité pouvant être victimes d'isolement physique, psychique, sensible et social, peuvent, en mettant en oeuvre la présente invention, contrevenir à cet isolement à travers des exercices visant à exercer la créativité et la capacité de communication. De plus, l'utilisateur patient peut retrouver et maîtriser la sensation de son corps et, notamment de son sens du toucher.

Des personnels spécialisés peuvent ainsi participer à des exercices réalisés par des patients.

Dans des modes de réalisation, la modulation d'un signal audio et/ou visuel comporte la modulation de la fréquence dudit signal. Par exemple, la fréquence sonore d'un son ou d'une oeuvre sonore est augmentée lorsque l'impédance du contact diminue. Par exemple, les longueurs d'ondes émises par une source de lumière diminuent avec l'impédance du contact. Ces variations rendent sensibles l'effet de variation du contact dermique.

Dans des modes de réalisation, la modulation d'un signal audio et/ou visuel comporte la modulation du rythme dudit signal. Par exemple, le rythme d'un battement régulier ou de restitution d'une œuvre sonore dépend de l'impédance du contact dermique.

Dans des modes de réalisation, le paramètre d'impédance détecté est la résistivité de la jonction.

Dans des modes de réalisation, le moyen de commande est déporté du détecteur et des électrodes, le dispositif comportant un moyen de transmission d'un signal sans-fil représentatif de la valeur détectée en direction du moyen de commande.

Ces modes de réalisation permettent de ne pas limiter le ou les utilisateurs dans leurs mouvements en raison de câbles de transmission les reliant au moyen de commande.

Dans des modes de réalisation, le dispositif comporte une source d'énergie électrique autonome configurée pour alimenter en courant électrique la première électrode.

Ces modes de réalisation permettent de ne pas limiter l'utilisateur dans ses mouvements en raison de câbles d'alimentation les reliant au moyen de commande.

Dans des modes de réalisation, le dispositif comporte un bracelet comportant la première électrode.

Ces modes de réalisation permettent de masquer l'électrode de la vue en conférant, de plus, à cette électrode un aspect plus plaisant.

Dans des modes de réalisation, le dispositif comporte une pluralité de première et/ou de deuxième électrodes.

Ces modes de réalisation permettent d'homogénéiser la capture ou la génération d'ondes dans la peau.

Dans des modes de réalisation, le moyen de commande commande l'émission d'un signal sonore dont un paramètre d'émission est déterminé en fonction de la valeur détectée.

Ces modes de réalisation permettent d'associer au sens du toucher le sens de l'audition.

Dans des modes de réalisation, le moyen de commande commande l'émission d'un signal lumineux dont un paramètre d'émission est déterminé en fonction de la valeur détectée.

Ces modes de réalisation permettent d'associer au sens du toucher le sens de la vue.

Dans des modes de réalisation, le moyen de commande commande l'émission d'un signal de déclenchement dans un média audiovisuel en cours de lecture.

Ces modes de réalisation permettent la commande d'une action dans un média interactif, tel un jeu vidéo par exemple, le dispositif agissant alors comme une interface homme-machine.

Dans des modes de réalisation, le moyen de commande commande l'émission d'un signal de modulation d'un média en cours de lecture.

Ces modes de réalisation permettent de moduler un média connu de l'utilisateur en fonction des contacts entre les peaux.

Dans des modes de réalisation, au moins une électrode est portée par une dalle sur laquelle un utilisateur pose au moins une partie de son corps. Ces modes de réalisation permettent de ne pas poser des électrodes sur la peau de l'utilisateur, celui-ci pouvant juste déplacer ou balancer ses pieds ou son corps sur des dalles pour bénéficier des effets de la présente invention.

Dans des modes de réalisation, la modulation d'un signal audio et/ou visuel dépend, de plus, de données physiologiques d'au moins un utilisateur, captées par le dispositif, le dispositif comportant au moins un capteur de données physiologique d'un utilisateur. Dans des modes de réalisation, au moins une donnée physiologique est une fréquence cardiaque. On enrichit, ainsi la sensation de son corps et de ses émotions par l'utilisateur.

### BREVE DESCRIPTION DES FIGURES

D'autres avantages, buts et caractéristiques particulières de l'invention ressortiront de la description non limitative qui suit d'au moins un mode de réalisation particulier de dispositifs et de procédés pour l'utilisation objet de la présente invention, en regard des dessins annexés, dans lesquels :
- les figures 1 et 2 représentent, schématiquement, des modes de réalisation particulier de dispositifs pour la mise en œuvre de l'invention,
- les figures 3 et 4 représentent, schématiquement, des médias modulés par la mise en œuvre de la présente invention,
- la figure 5 représente, schématiquement et sous forme d'un logigramme, une succession d'étapes particulière d'un procédé de modulation,
- la figure 6 représente, schématiquement, un premier mode de réalisation de l'utilisation objet de la présente invention,
- la figure 7 représente, schématiquement, un deuxième mode de réalisation de
   l'utilisation qui est présenté à titre d'exemple et n'est pas objet de la présente invention,
- la figure 8 représente, schématiquement, un troisième mode de réalisation de l'utilisation objet de la présente invention,
- la figure 9 représente, schématiquement, un quatrième mode de réalisation de
   l'utilisation qui est présenté à titre d'exemple et n'est pas objet de la présente invention,
- la figure 10 représente, schématiquement, un cinquième mode de réalisation de l'utilisation objet de la présente invention,
- la figure 11 représente, schématiquement, un sixième mode de réalisation de l'utilisation objet de la présente invention et
- la figure 12 représente, schématiquement, un septième mode de réalisation de l'utilisation objet de la présente invention.

### DESCRIPTION D'EXEMPLES DE REALISATION DE L'INVENTION

La présente description est donnée à titre non limitatif, chaque caractéristique d'un mode de réalisation pouvant être combinée à toute autre caractéristique de tout autre mode de réalisation de manière avantageuse.

On note dès à présent que les figures ne sont pas à l'échelle.

On appelle « terminal portable communicant » tout dispositif muni :
- d'une interface homme-machine et
- d'un moyen de communication de signaux filaires ou non, tels une antenne ou un port pour recevoir un câble réseau par exemple,

À titre d'exemple, un tel terminal portable communicant est :
- un ordiphone,
- une tablette numérique ou
- un ordinateur personnel.

Avant de décrire des dispositifs permettant la mise en œuvre de la présente invention, on donne, ci-après des explications sur différents modes de réalisation de l'utilisation de la modulation d'un signal audio et/ou visuel par une variation d'impédance d'au moins un contact avec la peau d'au moins un utilisateur pour l'entretien et/ou le développement de ses capacités physiques et/ou psychiques, utilisation mettant en œuvre de tels dispositifs.

Préférentiellement, cette utilisation utilise un flux continu de données représentatives du contact dermique, selon éventuellement, plusieurs paramètres :
- la zone de contact avec plus ou moins de capteurs sensoriels,
- la surface de contact (nombre de doigts, surface de la voute plantaire ou du dos) etc...
- la pression exercée sur ce contact,
- la température, un corps froid présentant une résistance électrique plus faible qu'un corps chaud,
- l'impédance, notamment en cas d'utilisation d'un tissu plus ou moins isolant (par exemple selon son humidité) ;

Le contrôle continu de l'impédance du contact dermique permet la pratique de l'utilisation selon l'invention, puisque c'est dans l'exploration des différences de valeurs, associées à la génération des sons ou de lumières, qui permet et suscite l'engagement de(s) utilisateur(s).

La pratique de synesthésie, lien entre les sens (le toucher et l'ouïe ou la vision), suscite l'engagement du corps physique (somatique), grâce au retour psychique (psyché), à travers la musique ou le spectacle visuel. Comme un véritable instrument de musique ou de création d'effets visuels, la valeur détectée génère le timbre, la hauteur, la longueur d'ondes, l'intensité et le rythme. Le geste faisant varier l'impédance est déductif car c'est l'écoute de la production sonore ou la vue des effets lumineux qui déterminent le geste à engager.

L'application est donc intimement liée puisque c'est elle qui permet la richesse des retours interactifs en temps réel : fréquences, enveloppes, volumes, combiné par paramètres de seuil de déclenchement, et interagissant en combinaison ou diffusion de fichiers audio numérique : gestion du volume et de la hauteur des sons d'ambiances (eau, mer, oiseaux), musiques préexistantes

L'invention apporte donc un outil de communication augmentée et une solution à un problème spécifique de communication des personnes handicapées. Elle permet à une personne paralysée d'être acteur avec le plus petit morceau de peau, à une personne autiste ou une personne aphasique de communiquer.

L'utilisateur, au-delà du divertissement, concentre ses sens comme pour toute pratique somatique (yoga, pleine conscience etc...), ce qui a, de plus, un effet relaxant.

L'invention fournit aussi un outil de rééducation, par la mobilisation physique. Par exemple, lorsque les électrodes sont au sol (par exemple, sous forme de plaques), les différences d'appui des pieds sont audibles comme différences de hauteur de son ; on travaille alors la proprioception.

L'invention fournit aussi un outil de rééducation, par la mobilisation psychique : la peau est certes un capteur sensoriel, mais aussi un outil de lien social, sans oublier la sexualité. La musique et les paysages sonores proposés (eau, vent, mer, rire d'enfants etc...) mobilisent la mémoire.

Ainsi, l'invention permet d'améliorer les capacités physiques et cognitives, et peut être mis à disposition des personnes souffrantes de troubles psychiatrique (dépression, burn out) mais aussi à destination des personnes âgées comme outil de prévention.

L'invention fournit aussi un outil au service des aidants professionnels et familiaux.

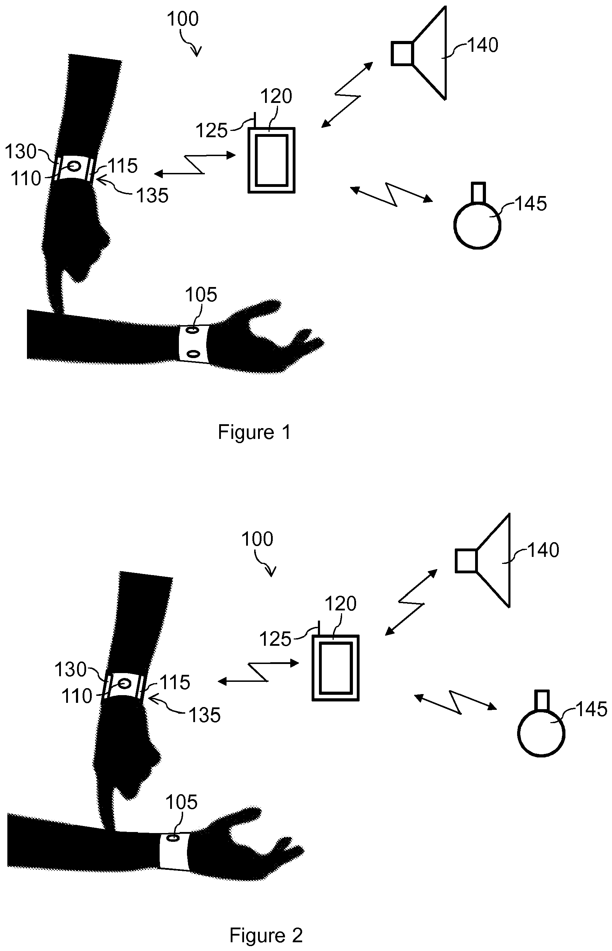

On observe, sur la figure 1, qui n'est pas à l'échelle, une vue schématique d'un mode de réalisation d'un dispositif 100 pour la mise en œuvre de la présente invention. Ce dispositif 100 de commande de diffusion d'un média comporte :
- une première électrode 105 dermique, configurée pour générer des ondes électriques, positionnée contre la peau d'un premier utilisateur,
- une deuxième électrode 110 dermique, configurée pour capter des ondes électriques, positionnée contre la peau d'un deuxième utilisateur, ce deuxième utilisateur pouvant être le premier utilisateur,
- un détecteur 115 de la valeur d'un paramètre d'impédance de la jonction, reliant les électrodes et passant par le contact avec la peau de chaque utilisateur et
- un moyen de 120 commande configuré pour émettre une commande de diffusion d'un média en fonction de la valeur détectée.

La première électrode 105 comporte, ou est relié à, un générateur d'ondes électriques de type oscillateur. Cet oscillateur est, de préférence, de type harmonique, c'est-à-dire configuré pour produite une onde de forme sinusoïdale. Cet oscillateur est, par exemple :
- oscillateur Colpitts,
- oscillateur Clapp,
- oscillateur à déphasage,
- oscillateur Pierce,
- oscillateur Hartley ou
- oscillateur à variables d'état.

Le courant électrique communiqué à la peau du premier utilisateur est, par exemple, d'une intensité de 1,5 milliampères et d'une tension de 9 Volts. Cette première électrode 105 présente, par exemple, un diamètre inférieur à deux centimètres.

Cette première électrode 105 est reliée à un moyen de commande du dispositif 100 configuré pour activer ou désactiver le générateur d'onde. Ce moyen de commande est, par exemple, un bouton poussoir ou un terminal portable communicant transmettant un signal sans-fil d'activation ou de désactivation du générateur d'onde.

Cette première électrode 105 est positionnée sur une dalle de grande taille par rapport aux dimensions de l'électrode 105. On entend, par « grande taille », une surface au moins cinq fois supérieure à la surface de la première électrode 105.

Dans des modes de réalisation préférentiels, tel que celui représenté en figure 1, le dispositif 100 comporte une pluralité de premières électrodes 105.

Les avantages de l'utilisation de systèmes multi-électrodes sont :
- de générer des commandes de diffusion indépendantes du même ou de différents média, et ainsi un enrichissement de la gamme média, signal sonore et/ou signal lumineux et/ou média audio-visuel et/ou média interactif et
- de pouvoir utiliser le dispositif 100 seul ou à plusieurs.

Les électrodes, 105 et 110, et le détecteur 115 peuvent être positionnés sur de grandes dalles, ce qui permet une utilisation polyphonique ou multimédia par le déplacement des pieds ou du corps sur cet ensemble de dalles indépendantes ou non.

Dans des variantes, au moins une électrode est positionnée contre une surface indépendante de l'utilisateur du système. Cette surface est, par exemple, un sol ou un mur. L'avantage d'un tel positionnement est de ne pas porter de bracelet, un tel port étant très difficile ou impossible à positionner sur des utilisateurs atteints de troubles du spectre autistique. Ainsi le contact du pied, en marchant ou de toute partie du corps effectue la jonction entre les électrodes. Le déplacement de dalle en dalle, seul ou à plusieurs, permet la génération d'une commande de diffusion du média.

Dans le cas où le dispositif 100 comporte plusieurs premières électrodes 105, chaque première électrode 105 peut être communicante indépendamment de chaque autre première 105 électrode. Dans des variantes, une seule première électrode 105 est communicante selon une liaison filaire ou sans-fil avec un dispositif distant et chaque autre première électrode 105 est reliée à la première électrode 105 communicante.

Dans des modes de réalisation préférentiels, tels que celui représenté en figure 1, le dispositif 100 comporte un bracelet 135 comportant chaque première 105 électrode.

Ce bracelet 135 est porté, par exemple, au poignet du premier utilisateur.

La deuxième électrode 110 comporte, ou est relié à, un capteur d'ondes électriques.

Cette deuxième électrode 110 est positionnée sur une dalle de grande taille par rapport aux dimensions de l'électrode 110. On entend, par « grande taille », une surface au moins cinq fois supérieure à la surface de la deuxième électrode 110.

Dans des modes de réalisation préférentiels (non représentés) le dispositif 100 comporte une pluralité de deuxièmes électrodes 110.

Dans le cas où le dispositif 100 comporte plusieurs deuxièmes électrodes 110, chaque deuxième électrode 110 peut être communicante indépendamment de chaque autre deuxième 110 électrode. Dans des variantes, une seule deuxième électrode 110 est communicante selon une liaison filaire ou sans-fil avec un dispositif distant et chaque autre deuxième électrode 110 est reliée à la deuxième électrode 110 communicante.

Dans des modes de réalisation préférentiels, tels que celui représenté en figure 1, le dispositif 100 comporte un bracelet (non référencé) comportant chaque deuxième 110 électrode.

Ce bracelet 135 est porté, par exemple, au poignet du deuxième utilisateur.

Ainsi, lorsque le premier utilisateur et le deuxième utilisateur sont en contact, les ondes générées par la première électrode 105 sont captées par la deuxième électrode 110.

Dans un mode de réalisation particulier, la première électrode 105 et la deuxième électrode 110 sont portées par un même utilisateur, et le contact d'une première partie du corps avec une deuxième partie du corps a pour effet de changer le chemin parcouru par les ondes dans le corps.

Dans un état d'absence de contact, où chaque électrode, 105 et 110, est positionnée sur un poignet différent de l'utilisateur, les ondes sont transmises à travers les bras et le torse de l'utilisateur.

Dès que l'utilisateur touche, avec l'une de ses mains, l'autre avant-bras, le chemin parcouru par les ondes devient : une partie de bras, main puis une autre partie de bras jusqu'au capteur, ce chemin étant plus court que le chemin en état d'absence de contact.

On observe, en figures 1 et 2, deux positionnements différents du contact entre les utilisateurs, ces positionnements entraînant des valeurs de paramètre d'impédance différents.

Le détecteur 115 est, par exemple, un circuit électrique configuré pour comparer l'onde électrique émise et l'onde électrique captée pour déterminer, par exemple :
- la résistivité,
- la conductivité,
- la résistance,
- l'impédance et/ou
- la capacité

de la jonction reliant la première et la deuxième électrode, 105 et 110 et passant par le contact avec la peau de chaque utilisateur.

Ainsi, chacun de ces paramètres change en fonction du positionnement du contact entre les utilisateurs, de la pression exercée au niveau de ce contact et de la surface de ce contact.

La valeur du paramètre détectée est transmise au moyen de commande 120. Le détecteur 115 commande est positionnés :
- soit à proximité des électrodes, pour être portés par l'utilisateur, et transportons le son par transmission HF,
- soit au niveau d'un terminal portable communicant relié de manière filaire aux électrodes, 105 et 110.

Le moyen de commande 120 est, par exemple, un circuit électronique embarqué dans un terminal communicant, ce circuit électronique émettant des commandes en direction :
- d'un transducteur 140 électroacoustique,
- d'un émetteur 145 de signaux lumineux et/ou
- d'un périphérique de lecture d'un média audiovisuel.

Ce moyen de commande 120 comporte, de préférence, un programme informatique de pilotage du circuit électronique, ce programme informatique étant configuré pour :
- acquérir le signal capté par le détecteur 115 via la deuxième électrode 110,
- normaliser et lisser ce signal,
- matricer ce signal normalisé et lissé,
- déterminer :
   - des seuils de déclenchement d'événements,
   - des contrôles continus sur des paramètres de synthèse :
      - par échantillonnage,
      - par modulation de fréquence,
      - par synthèse additive et
- mixer le signal à lire.

Ce moyen de commande 120 émet une commande qui dépend de la valeur du paramètre transmise.

Par exemple, le moyen de commande 120 émet une commande d'émission d'un son à une fréquence dont la valeur est déterminée en fonction de la valeur du paramètre transmise. Par exemple, plus la résistivité détectée est élevée, plus la fréquence d'émission ou l'amplitude d'un signal sonore est élevée. Ce son peut être polyphonique.

Dans des modes de réalisation, le moyen 120 de commande commande l'émission d'un signal sonore dont un paramètre d'émission est déterminé en fonction de la valeur détectée.

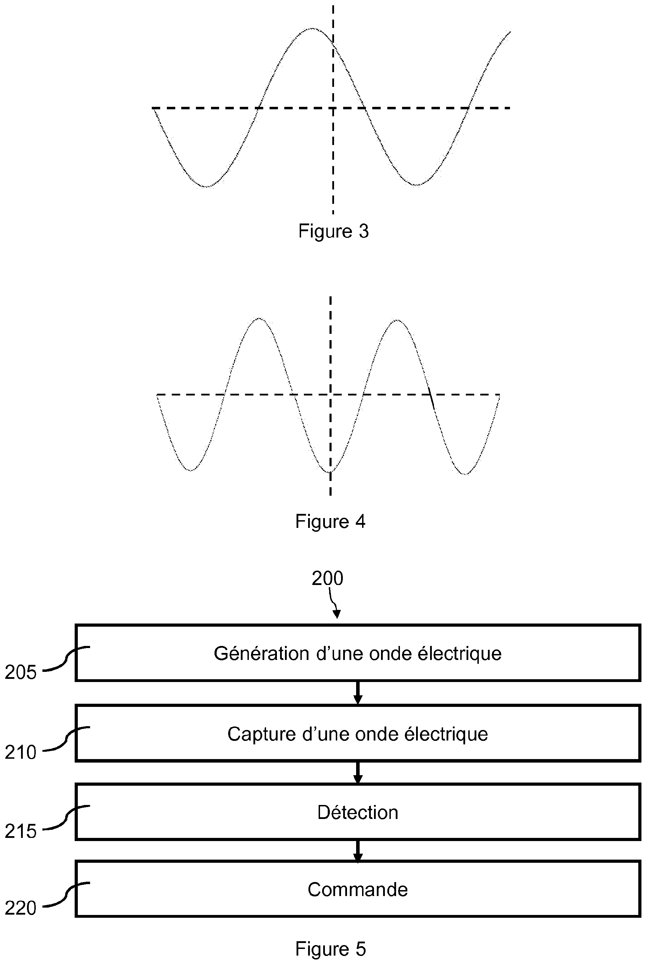

On observe, notamment en figure 3 et 4, un signal sinusoïdal correspondant à un son émis par un transducteur électroacoustique. La fréquence de ce signal correspond, en figure 3, au positionnement du contact illustré en figure 1 tandis que la fréquence du signal, en figure 4, correspond au positionnement du contact illustré en figure 2. On comprend ainsi que le raccourcissement du chemin reliant les électrodes entraîne une augmentation de la fréquence.

Dans des modes de réalisation, le moyen 120 de commande commande l'émission d'un signal lumineux dont un paramètre d'émission est déterminé en fonction de la valeur détectée.

On appel « signal lumineux » tout signal produit par une source d'émission de lumière. Un tel signal lumineux est, par exemple, un signal émis par une diode, un écran ou un vidéoprojecteur.

Dans des modes de réalisation, le moyen 120 de commande commande l'émission d'un signal de déclenchement dans un média audiovisuel en cours de lecture.

Ce signal de déclenchement est, par exemple, une commande d'interruption, de lecture, d'augmentation ou de réduction du volume sonore, ou une commande correspondant habituellement à la pression d'une touche d'un clavier.

Dans des modes de réalisation, le moyen 120 de commande commande l'émission d'un signal de modulation d'un média en cours de lecture.

Le signal de modulation est, par exemple, un signal de modulation d'un signal sonore ou visuel pour modifier un paramètre de lecture du média.

Dans des modes de réalisation, tels que celui représenté en figure 1, le moyen 120 de commande est déporté du détecteur 115 et des électrodes 105, 110, le dispositif comportant un moyen 125 de transmission d'un signal sans-fil représentatif de la valeur détectée en direction du moyen de commande.

Le moyen de transmission 125 est, par exemple, une antenne associée au moyen de commande 120 configurée pour émettre et recevoir des signaux sans-fil selon la norme Bluetooth (Marque déposée), selon le standard IEEE 802.11 dit « Wi-Fi », et plus généralement selon une technologie de transmission à hautes fréquences par exemple.

Dans des modes de réalisation, tels que celui représenté en figure 1, le dispositif 100 comporte une source 130 d'énergie électrique autonome configurée pour alimenter en courant électrique la première électrode 105.

Cette source d'énergie 130 est, par exemple, une pile bouton.

Dans des modes de réalisation, le dispositif 100 comporte, de plus, un capteur de données physiologiques, par exemple un cardio-fréquencemètre. La valeur des données physiologiques mesurée, par exemple la fréquence cardiaque, est combinée mathématiquement avec l'impédance du contact dermique pour moduler la génération ou la diffusion de signaux sonores et/ou lumineux.

On observe, sur la figure 5, un mode de réalisation particulier du procédé 200 permettant l'utilisation objet de la présente invention. Ce procédé 200 de commande de diffusion d'un média comporte :
- une étape 205 de génération d'ondes électriques transmises à la peau d'un premier utilisateur au moyen d'une première électrode,
- une étape 210 de capture d'ondes électriques transmises par la peau d'un deuxième utilisateur au moyen d'une deuxième électrode, ce deuxième utilisateur pouvant être le premier utilisateur,
- une étape de détection 215 de la valeur d'un paramètre d'impédance de la jonction reliant les électrodes et
- une étape de 220 commande configuré pour émettre une commande de diffusion d'un média en fonction de la valeur détectée.

Dans des modes de réalisation, au cours de l'étape 220, la modulation d'un signal audio et/ou visuel dépend, de plus, de données physiologiques d'au moins un utilisateur, captées par le dispositif, le dispositif comportant au moins un capteur de données physiologique d'un utilisateur. Par exemple, au moins une donnée physiologique est une fréquence cardiaque.

Ce procédé 200 est mis en œuvre, par exemple, par l'utilisation d'un dispositif 100 tel que décrit en regard des figures 1 et 2.

Préférentiellement, la modulation d'un signal audio et/ou visuel comporte la modulation de la fréquence dudit signal. Par exemple, la fréquence sonore d'un son ou d'une œuvre sonore est augmentée lorsque l'impédance du contact diminue. Selon un autre exemple, les longueurs d'ondes émises par une source de lumière diminuent avec l'impédance du contact. Ces variations rendent sensibles l'effet de variation du contact dermique.

Dans des modes de réalisation, la modulation d'un signal audio et/ou visuel comporte la modulation du rythme dudit signal. Par exemple, le rythme d'un battement régulier ou de restitution d'une œuvre sonore dépend de l'impédance du contact dermique.

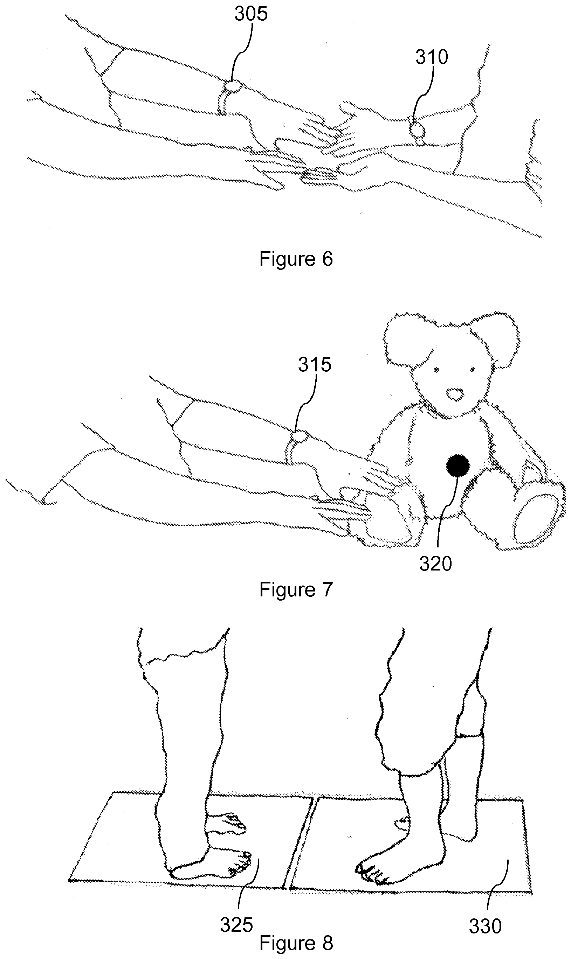

On observe, en figure 6, schématiquement, un premier mode de réalisation de l'utilisation objet de la présente invention, dans lequel chacune de deux personnes, par exemple un patient et un aidant, porte un bracelet, respectivement 305 et 310, comportant une électrode. Optionnellement, au moins l'un des bracelets, préférentiellement associé à l'électrode de capture de signaux électriques, comporte un cardio-fréquencemètre (non représenté). Selon l'impédance du contact entre les deux utilisateurs, ici représenté par un contact entre les mains des utilisateurs, et optionnellement selon la fréquence cardiaque d'au moins un des utilisateurs, on module l'émission d'un signal sonore et/ou lumineux, voire audiovisuel ou on module la diffusion d'un média audio-visuel. Ainsi, par retour sur au moins un des sens de l'ouïe ou de la vue, chaque utilisateur peut ressentir et maîtriser le contact et faire varier les effets ressentis.

On observe, en figure 7, un deuxième mode de réalisation de l'utilisation qui est présenté à titre d'exemple et n'est pas objet de la présente invention, dans lequel un objet, ici un ours en matériau électriquement conducteur, porte une électrode 320. Une personne, par exemple un patient, porte un bracelet comportant une électrode 315. Optionnellement, le bracelet, préférentiellement associé à l'électrode de capture de signaux électriques, comporte un cardio-fréquencemètre (non représenté). Selon l'impédance du contact entre l'utilisateur et l'objet, et optionnellement selon la fréquence cardiaque d'au moins un des utilisateurs, on module l'émission d'un signal sonore et/ou lumineux, voire audiovisuel ou on module la diffusion d'un média audio-visuel. Ainsi, par retour sur au moins un des sens de l'ouïe ou de la vue, l'utilisateur peut ressentir et maîtriser le contact et faire varier les effets ressentis.

Ainsi, comme on le comprend à la lecture de la description des figures 6 et 7, dans des modes de réalisation, la modulation d'un signal audio et/ou visuel dépend, de plus, de données physiologiques d'au moins un utilisateur, captées par le dispositif, le dispositif comportant au moins un capteur de données physiologique d'un utilisateur. Par exemple, au moins une donnée physiologique est une fréquence cardiaque.

On observe, en figure 8, schématiquement, un troisième mode de réalisation de l'utilisation objet de la présente invention, dans lequel deux utilisateurs ont leurs pieds nus sur deux dalles 325 et 330 munies, chacune d'une électrode. Selon l'impédance du contact entre les deux utilisateurs, par exemple un contact entre les bras ou les mains des utilisateurs, on module l'émission d'un signal sonore et/ou lumineux, voire audiovisuel ou on module la diffusion d'un média audio-visuel. Ainsi, par retour sur au moins un des sens de l'ouïe ou de la vue, chaque utilisateur peut ressentir et maîtriser le contact et faire varier les effets ressentis.

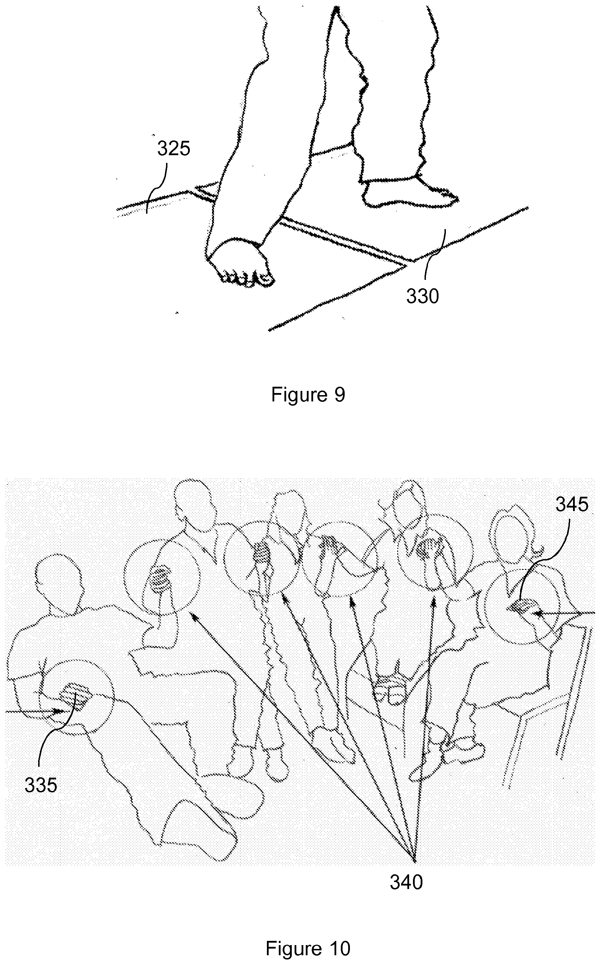

On observe, en figure 9, schématiquement, un quatrième mode de réalisation de l'utilisation qui est présenté à titre d'exemple et n'est pas objet de la présente invention, dans lequel un utilisateur pose ses pieds nus sur les dalles 325 et 330.

On observe, en figure 10, schématiquement, un cinquième mode de réalisation de l'utilisation objet de la présente invention, dans lequel deux utilisateurs sont munis, chacun, d'une électrode, respectivement 335 et 345, par exemple par l'intermédiaire d'un bracelet. Les zones de contact 340 entre deux utilisateurs successifs d'une chaîne d'utilisateurs, ici cinq, influencent l'impédance totale entre les électrodes 335 et 345.

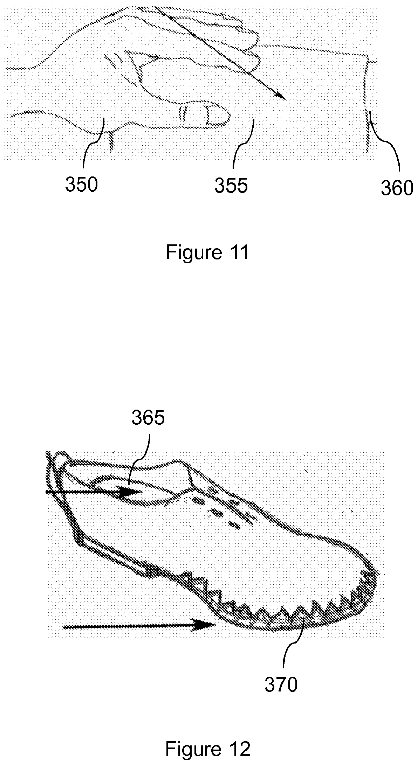

On observe, en figure 11, schématiquement, un sixième mode de réalisation de l'utilisation objet de la présente invention, dans lequel un tissu 355 est interposé entre les mains 350 et 360 de deux utilisateurs. Ce tissu, plus ou moins sec ou humide, diminue ou augmente l'impédance du contact entre les mains. On note que les mains considérées peuvent être celles d'un seul utilisateur ou de deux utilisateurs différents.

On observe, en figure 12, schématiquement, un septième mode de réalisation de l'utilisation objet de la présente invention, dans lequel une chaussure électriquement isolante comporte une électrode interne 365 et une électrode externe 370. Le contact électrique réalisé par le reste du corps, par exemple au contact d'un arbre ou du sol, formant ainsi un circuit conducteur entre les électrodes, module l'impédance du contact avec le corps de l'utilisateur.

Dans chacun de ces modes de réalisation, on utilise la modulation d'un signal audio et/ou visuel par une variation d'impédance d'au moins un contact avec la peau d'au moins un utilisateur pour l'entretien et/ou le développement de ses capacités physiques et/ou psychiques, en mettant en œuvre un dispositif tel que décrit en regard des figures 1 à 4.

## Revendications

1. Utilisation de la modulation d'un signal audio et/ou visuel par une valeur de paramètre d'impédance d'une jonction électrique comportant au moins un contact avec la peau d'au moins un utilisateur pour l'entretien et/ou le développement de ses capacités physiques et/ou psychiques, utilisation mettant en oeuvre un dispositif (100) de commande de diffusion d'un média, qui comporte :
- une première électrode (105) dermique, configurée pour générer des ondes électriques, positionnée contre la peau d'un premier utilisateur,
- une deuxième électrode (110) dermique, configurée pour capter des ondes électriques, positionnée contre la peau d'un deuxième utilisateur, le premier et le deuxième utilisateurs étant deux utilisateurs différents,
- un détecteur (115) de la valeur d'un paramètre d'impédance de la jonction reliant les électrodes passant par le contact avec la peau de chaque utilisateur et
- un moyen (120) de commande configuré pour émettre une commande de modulation de diffusion d'un média représentant le signal audio et/ou visuel en fonction de la valeur détectée ;
dans laquelle la jonction pour laquelle une valeur de paramètre d'impédance est détecté comporte au moins un contact entre deux utilisateurs différents.

2. Utilisation selon la revendication 1, dans laquelle la modulation d'un signal audio et/ou visuel comporte la modulation de la fréquence dudit signal.

3. Utilisation selon l'une des revendications 1 ou 2, dans laquelle la modulation d'un signal audio et/ou visuel comporte la modulation d'un rythme de restitution dudit signal.

4. Utilisation selon l'une des revendications 1 à 3, dans laquelle la valeur de paramètre d'impédance détecté est la résistivité de la jonction.

5. Utilisation selon l'une des revendications 1 à 4, dans laquelle le moyen (120) de commande est déporté du détecteur (115) et des électrodes (105, 110), le dispositif comportant un moyen (125) de transmission d'un signal sans-fil représentatif de la valeur détectée en direction du moyen de commande.

6. Utilisation selon l'une des revendications 1 à 5, dans laquelle le dispositif (100) comporte une source (130) d'énergie électrique autonome configurée pour alimenter en courant électrique la première électrode (105).

7. Utilisation selon l'une des revendications 1 à 6, dans laquelle le dispositif (100) comporte un bracelet (135) comportant la première (105) électrode.

8. Utilisation selon l'une des revendications 1 à 7, dans laquelle le dispositif (100) comporte une pluralité de première et/ou de deuxième électrodes (105, 110).

9. Utilisation selon l'une des revendications 1 à 8, dans laquelle le moyen (120) de commande module la diffusion d'un média en commandant l'émission d'un signal sonore du média, signal sonore dont un paramètre d'émission est déterminé en fonction de la valeur détectée.

10. Utilisation selon l'une des revendications 1 à 9, dans laquelle le moyen (120) de commande module la diffusion d'un média en commandant l'émission d'un signal lumineux du média, signal lumineux dont un paramètre d'émission est déterminé en fonction de la valeur détectée.

11. Utilisation selon l'une des revendications 1 à 10, dans laquelle le moyen (120) de commande module la diffusion d'un média audiovisuel en commandant l'émission d'un signal de déclenchement dans un média audiovisuel en cours de lecture.

12. Utilisation selon l'une des revendications 1 à 11, dans laquelle le moyen (120) de commande module la diffusion d'un média en commandant l'émission d'un signal de modulation d'un média en cours de lecture.

13. Utilisation selon l'une des revendications 1 à 12, dans laquelle au moins une électrode est portée par une dalle sur laquelle un utilisateur pose au moins une partie de son corps.

14. Utilisation selon l'une des revendications 1 à 13, dans laquelle, la modulation d'un signal audio et/ou visuel dépend, de plus, de données physiologiques d'au moins un utilisateur, captées par le dispositif, le dispositif comportant au moins un capteur de données physiologique d'un utilisateur.

## Patentansprüche

1. Verwendung der Modulation eines akustischen und/oder visuellen Signals durch einen Impedanzparameterwert einer elektrischen Verbindung, aufweisend mindestens einen Kontakt mit der Haut von mindestens einem Benutzer zur Erhaltung und/oder Entwicklung seiner physischen und/oder psychischen Fähigkeiten, wobei die Verwendung eine Vorrichtung (100) zur Steuerung der Verbreitung eines Mediums umsetzt, die aufweist:
- eine erste, zum Erzeugen von elektrischen Wellen ausgelegte Dermalelektrode (105), die auf der Haut eines ersten Benutzers positioniert ist,
- eine zweite, zum Einfangen von elektrischen Wellen ausgelegte Dermalelektrode (110), die auf der Haut eines zweiten Benutzers positioniert ist, wobei der erste und der zweite Benutzer zwei verschiedene Benutzer sind,
- einen Detektor (115) des Werts eines Impedanzparameters der die Elektroden verbindenden Verbindung, die durch den Kontakt mit der Haut jedes Benutzers erfolgt, und
- ein Steuermittel (120), das ausgelegt ist, um einen Befehl zur Modulation der Verteilung eines das akustische und/oder visuelle Signal darstellenden Mediums in Abhängigkeit von dem ermittelten Wert zu senden;
wobei die Verbindung, für die ein Impedanzparameterwert ermittelt wird, mindestens einen Kontakt zwischen zwei verschiedenen Benutzern aufweist.

2. Verwendung nach Anspruch 1, wobei die Modulation eines akustischen und/oder visuellen Signals die Modulation der Frequenz des Signals aufweist.

3. Verwendung nach einem der Ansprüche 1 oder 2, wobei die Modulation eines akustischen und/oder visuellen Signals die Modulation eines Wiedergaberhythmus des Signals aufweist.

4. Verwendung nach einem der Ansprüche 1 bis 3, wobei der ermittelte Impedanzparameterwert die Resistivität der Verbindung ist.

5. Verwendung nach einem der Ansprüche 1 bis 4, wobei das Steuermittel (120) vom Detektor (115) und den Elektroden (105, 110) versetzt ist, wobei die Vorrichtung ein drahtloses Übertragungsmittel (125) eines Signals, das für den ermittelten Wert repräsentativ ist, in Richtung des Steuermittels aufweist.

6. Verwendung nach einem der Ansprüche 1 bis 5, wobei die Vorrichtung (100) eine autonome Elektroenergiequelle (130) aufweist, die ausgelegt ist, um die erste Elektrode (105) mit elektrischem Strom zu versorgen.

7. Verwendung nach einem der Ansprüche 1 bis 6, wobei die Vorrichtung (100) ein Armband (135) aufweist, das die erste (105) Elektrode aufweist.

8. Verwendung nach einem der Ansprüche 1 bis 7, wobei die Vorrichtung (100) eine Vielzahl von ersten und/oder zweiten Elektroden (105, 110) aufweist.

9. Verwendung nach einem der Ansprüche 1 bis 8, wobei das Steuermittel (120) die Verbreitung eines Mediums durch Steuern des Sendens eines akustischen Signals des Mediums moduliert, wobei ein Sendeparameter des akustischen Signals in Abhängigkeit von dem ermittelten Wert bestimmt wird.

10. Verwendung nach einem der Ansprüche 1 bis 9, wobei das Steuermittel (120) die Verbreitung eines Mediums durch Steuern des Sendens eines Lichtsignals des Mediums moduliert, wobei ein Sendeparameter des Lichtsignals in Abhängigkeit von dem ermittelten Wert bestimmt wird.

11. Verwendung nach einem der Ansprüche 1 bis 10, wobei das Steuermittel (120) die Verbreitung eines audiovisuellen Mediums durch Steuern des Sendens eines Auslösesignals in einem audiovisuellen Medium während des Lesens moduliert.

12. Verwendung nach einem der Ansprüche 1 bis 11, wobei das Steuermittel (120) die Verbreitung eines Mediums durch Steuern des Sendens eines Modulationssignals eines Mediums während des Lesens moduliert.

13. Verwendung nach einem der Ansprüche 1 bis 12, wobei mindestens eine Elektrode von einer Platte getragen wird, auf die ein Benutzer mindestens einen Teil seines Körpers stellt.

14. Verwendung nach einem der Ansprüche 1 bis 13, wobei die Modulation eines akustischen und/oder visuellen Signals weiterhin von physiologischen Daten mindestens eines Benutzers abhängt, die von der Vorrichtung erfasst werden, wobei die Vorrichtung mindestens einen Sensor physiologischer Daten eines Benutzers aufweist.

## Claims

1. Use of the modulation of an audio and/or visual signal by a value of an impedance parameter of at least one electric junction comprising at least one contact with the skin of at least one user to maintain and/or develop the user's physical and/or mental abilities, utilizing a device (100) for controlling the delivery of a medium, said device comprising:
- a first skin electrode (105) configured to generate electric waves, positioned against the skin of a first user;
- a second skin electrode (110) configured to capture electric waves, positioned against the skin of a second user, the first and the second user being two different users;
- a detector (115) for detecting the value of an impedance parameter of the junction connecting the electrodes and passing through the contact with the skin of each user; and
- a control means (120) configured to transmit a modulated medium-delivery command representing the audio and/or visual signal according to the value detected
wherein the junction for which a value of an impedance parameter is detected comprises at least one contact between two different users.

2. Use according to claim 1, wherein the modulation of an audio and/or visual signal comprises the modulation of the frequency of said signal.

3. Use according to claims 1 or 2, wherein the modulation of an audio and/or visual signal comprises the modulation of the rhythm of said signal.

4. Use according to one of claims 1 to 3, wherein the impedance parameter detected is the resistivity of the junction.

5. Use according to one of claims 1 to 4, wherein the control means (120) is separate from the detector (115) and electrodes (105, 110), the device comprising a means (125) for transmitting a wireless signal representative of the value detected towards the control means.

6. Use according to one of claims 1 to 5, wherein the device (100) comprises an independent source of electrical power (130), configured to supply the first electrode (105) with electrical current.

7. Use according to one of claims 1 to 6, wherein the device (100) comprises a bracelet (135) comprising the first electrode (105).

8. Use according to one of claims 1 to 2, wherein the device (100) comprises a plurality of first and/or second electrodes (105, 110).

9. Use according to one of claims 1 to 8, wherein the control means (120) controls the emission of a sound signal for which an emission parameter is determined as a function of the value detected.

10. Use according to one of claims 1 to 9, wherein the control means (120) controls the emission of a light signal for which an emission parameter is determined as a function of the value detected.

11. Use according to one of claims 1 to 10, wherein the control means (120) controls the emission of a trigger signal in an audiovisual medium being played.

12. Use according to one of claims 1 to 11, wherein the control means (120) controls the emission of a modulation signal for a medium being played.

13. Use according to one of claims 1 to 12, wherein at least one electrode is carried by a panel on which the user places at least one part of his body.

14. Use according to one of claims 1 to 13, wherein the modulation of an audio and/or visual signal also depends on physiological data of at least one user, captured by the device, the device comprising at least one sensor of a user's physiological data.
